# EUROPEAN PATENT APPLICATION

(11) **EP 2 574 921 A1**
(43) Date of publication of application: **03.04.2013**
(21) Application number: 11183133.5
(22) Date of filing: 28.09.2011
(51) Int. Cl.: G01N 33/44, G01N 7/14

(54) **Method and apparatus for determining the amount of volatile impurities in a liquid material**

(71) Applicant: Siemens Aktiengesellschaft, 80333 München (DE)
(72) Inventor: Grove-Nielsen, Erik, 7870 Roslev (DK); Wolf, Erik, 91341 Röttenbach (DE)

(57) **Abstract**

The method comprises the steps of filling a measuring chamber completely with a liquid material (100), closing the measuring chamber hermetically sealed (110), reducing the pressure in the measuring chamber to a predefined pressure such that the volatile impurities are released over a free surface of the liquid material as gas bubbles (120), maintaining this pressure for a predetermined time such that the gas bubbles collapse leaving a gas volume, and measuring the gas volume (130) which indicates the amount of volatile impurities.

Furthermore, an apparatus for carrying out this method is suggested comprising a hermetically sealable measuring chamber (8) with a first opening (1) for introducing a liquid material (11) and a second opening (2) for connecting a vacuum generator. The apparatus further comprises a transparent panel (9) for measuring the gas volume resulting from released volatile impurities.

## Description

The invention relates to a method and an apparatus for determining the amount of volatile impurities in a liquid material.

It is often desirable to determine the amount of volatile impurities in a liquid material, for instance the air content in a polymer material.

A common process for manufacturing fibre reinforced composite products is the vacuum assisted resin transfer moulding (VARTM). During the VARTM process, fabrics are placed in a mould, the mould is sealed and evacuated. Then, wet resin is injected into the mould and sucked into a cavity under vacuum. Although this process is cost-saving and allows precise tolerances and detailed shaping, it can sometimes fail to fully saturate the fabrics leading to weak spots in the final shape.

Air voids or other gas voids are released in the uncured resin at low pressure which leads to lower physical strength and lower overall quality of the final component, which in turn necessitates costly repair and refinishing work.

Therefore, it is desirable to determine the amount of air voids and other gas voids in a liquid material prior to the VARTM or other production processes.

Internally, it is known to cast test panels and to evaluate the quality of the material by measuring the density of the test panels prior to a casting process of composite components. This method demands a lot of time and effort.

It is therefore an object of the invention to provide a method for determining the amount of volatile impurities in a liquid material which is less time consuming compared to determination methods known in the art. It is a further object of the present invention to provide an apparatus for determining the amount of volatile impurities in a liquid material which is easily applicable under production process conditions and efficient.

The aim of the invention is achieved by the features of the independent claims.

Further aspects of the invention are subject of the dependent claims.

A method for determining the amount of volatile impurities in a liquid material comprises the steps of introducing a liquid material comprising volatile impurities into a measuring chamber, closing the measuring chamber hermetically sealed, reducing the pressure in the measuring chamber to a predefined pressure such that the volatile impurities are released over a free surface of the liquid material as gas bubbles, maintaining the predefined pressure in the measuring chamber for a predetermined time such that the gas bubbles collapse leaving a gas volume, and measuring the gas volume which indicates the amount of volatile impurities in the liquid material.

The measuring chamber is completely filled with the liquid material. This also ensures unchanged measurement conditions when measuring a series of liquid material samples or different liquid materials. Hence, the results of these measurements may be compared easily.

The liquid material may comprise a liquid plastic material for instance a polymeric material like a thermosetting polymer. Hence, the method may be applied in a production process of fiber reinforced composite components to evaluate the quality of a polymer melt prior to a casting process. A polymer material of a lower quality may be identified and rejected which enhances the quality of the final composite component. The overall production costs are reduced, which is especially advantageous in case of expensive and laborious processes, for instance casting processes of wind turbine rotor blades.

Furthermore, the liquid material may be heated in the measuring chamber to reduce the viscosity of the liquid material to facilitate releasing of gas bubbles.

An apparatus for determining the amount of volatile impurities in a liquid material comprises a measuring chamber with a bottom part, a chamber part and a measuring part. The bottom part is connected to the chamber part which in turn is connected to the measuring part such that these parts enclose the measuring chamber.

The bottom part comprises a first closable opening for introducing a liquid material having volatile impurities into the measuring chamber. The bottom part further comprises a second closable opening connectable to a vacuum generator for reducing the pressure in the measuring chamber for a predetermined time such that the volatile impurities are released from the liquid material as gas bubbles, wherein the gas bubbles rise to a free surface of the liquid material.

By arranging the second closable opening in the bottom part, it is avoided that gas bubbles are discharged from the measuring chamber when a vacuum is applied. Even a high vacuum may be applied through the second opening in the bottom part without sucking off gas bubbles from the measuring chamber.

A third closable opening may be arranged in the measuring chamber, preferably at the top of the measuring chamber. This third closable opening may be used to evacuate the measuring chamber and to suck the liquid material into the measuring chamber through the first closable opening.

Furthermore, the measuring chamber comprises one or more transparent panels for oberserving the processes going on in the measuring chamber and in particular, for measuring a gas volume resulting from gas bubbles released over a free surface of the liquid material. For this purpose a measuring scale may be arranged at the one or more transparent panels. Hence, the apparatus is effective and nevertheless cost-efficient and robust.

In an embodiment of the invention, the measuring part comprises a tubular part and the chamber part comprises a cone-shaped part. Although, these parts may have other shapes, a tubular measuring part and an at least partly cone-shaped chamber part allow for determining the amount of volatile impurities more precisely. This is because the cone-shaped form supports assembling and rising of gas bubbles released from the liquid material and the tubular form of the measuring part facilitates the measurement of the gas volume.

Besides, the apparatus may further comprise at least one sensing device which allows for detecting the filling degree of the measuring chamber, for sensing the vacuum conditions in the measuring chamber or for measuring other process values.

In the following, the invention will be described by way of example in more detail.

The drawings show preferred configurations and do not limit the scope of the invention. The same reference numerals are used in the drawings for elements having the same function.
**FIG 1** shows the method in a flow chart
**FIG 2** shows schematically an apparatus according to an embodiment of the invention connectable to a vacuum generator
**FIG 3a, 3b, 3c** show schematically the method using the apparatus shown in FIG 2
**FIG 4** shows a measurement setup comprising an apparatus 21 according to an embodiment of the invention

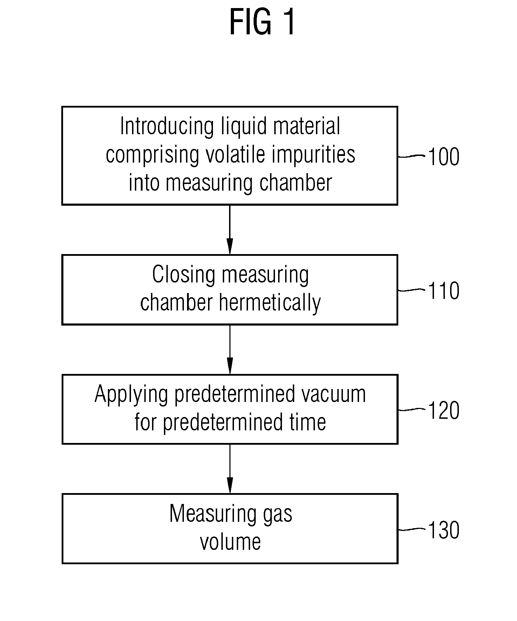

**FIG 1** shows in a flow chart an example of the method for determining the amount of volatile impurities in a liquid material.

Although the method and apparatus herein after explained may be used to determine the amount of volatile impurities of various liquid materials, like plastic or glass materials, they will be described in the following using a liquid polymer as example of such a liquid material.

Volatile impurities in a liquid polymer comprise gaseous components, for instance trapped air. Furthermore, the volatile impurities may comprise liquid components which volatilize, if the material is exposed to a vacuum, i.e. to a pressure which is below the atmospheric pressure of 1000 mbar. This happens for instance during a casting process of a composite component. An example of such a liquid component in this context is a reactive diluent in an epoxide resin.

As shown in **FIG 1****,** the method comprises the step of filling a measuring chamber completely with a liquid polymer comprising volatile impurities 100.

Then, the measuring chamber is closed hermetically 110, wherein "hermetically" or "hermetically sealed" means leakproof and vacuum tight in such a way that a vacuum may be applied to the measuring chamber. Thereby, applying a vacuum may involve discharging a small portion of the liquid material from the measuring chamber.

After having closed the measuring chamber, the pressure in the measuring chamber is reduced to a predefined pressure such that the volatile impurities are released from the liquid polymer as gas bubbles. The predefined pressure is maintained for a predetermined time 120. During this time, gas bubbles in the liquid material expand, migrate into other gas bubbles, assemble and rise upwardly. After some time, the gas bubbles are released over a free surface of the liquid polymer in the measuring chamber and thus, separated from the liquid polymer. Finally, the gas bubbles collapse and leave behind a gas volume which is measured 130, for instance by reading of a free gas column in a tubular part of the measuring chamber.

The gas volume is measured 130 after a predetermined time. The predetermined time may also be referred to as waiting time and depends on the liquid material to be evaluated and on the temperature of the liquid material.

The step of filling 100 a measuring chamber completely with a liquid polymer comprising volatile impurities may include applying a low vacuum to the measuring chamber for sucking the liquid polymer into the measuring chamber. Alternatively, the liquid polymer may be injected into the measuring chamber.

A low vacuum, for instance in the range of 800 mbar to 950 mbar, is sufficient to suck the liquid polymer from a container into the measuring chamber.

Furthermore, the liquid polymer may be heated, for instance to a temperature in the range of 40 to 80 degrees Celsius, to reduce the viscosity of the polymer. Thereby, the liquid polymer is heated before being introduced into the measuring chamber or in the measuring chamber. A combination of both is also possible, i.e. preheating the material, introducing the material into the measuring chamber and then, heating the measuring chamber.

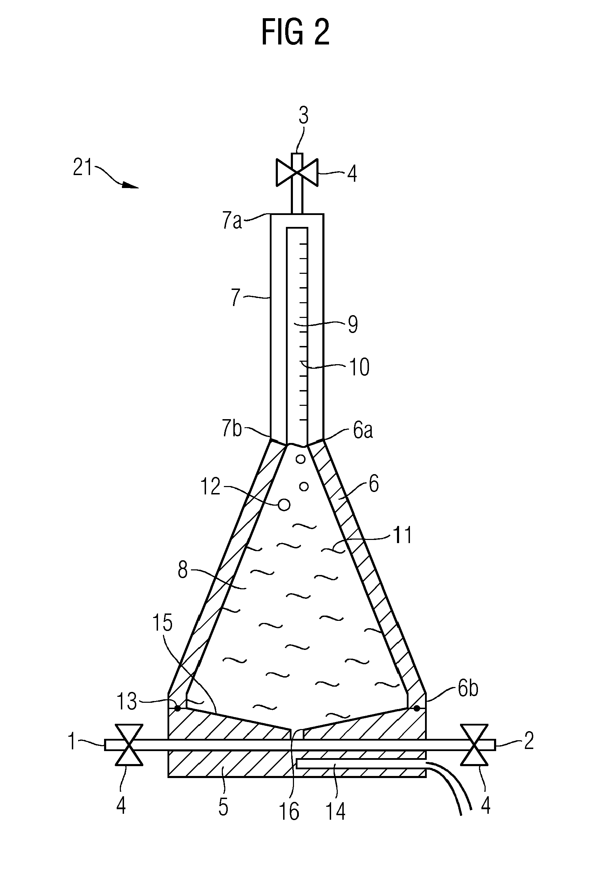

In the following, an apparatus 21 for carrying out the aforementioned method will be described in more detail, referring to the figures, especially to **FIG 2****.**

The apparatus 21 comprises a hermetically sealable measuring chamber 8 with a bottom part 5, a chamber part 6 and a measuring part 7, wherein the bottom part 5 is connected to the chamber part 6 and the chamber part 6 is connected to the measuring part 7.

The embodiment, shown in FIG 2, comprises a cone-shaped chamber part 6 and a tubular measuring part 7. The tubular measuring part 7 and the cone-shaped chamber part 6 form a single funnel-shaped piece. That means, the cone-shaped chamber part 6 comprises a tapered end 6a to which an end of the tubular measuring part 7b is connected.

As further shown in FIG 2, the measuring chamber 8 comprises a cylindrical bottom part 5 which is connected to the cone-shaped chamber part 6. A sealing 13, for instance an o-ring, is arranged between an end of the cone-shaped chamber part 6b and the bottom part 5. The sealing 13 may be made of rubber or any other suitable sealing material.

The bottom part 5 further comprises an inlet port 1 for introducing a liquid polymer 11 comprising volatile impurities into the measuring chamber 8. Preferably, the inlet port 1 is furnished with a valve 4, for instance a solenoid valve, for closing the inlet port 1 after having introduced the liquid polymer 11. The inlet opening 1 is also used as outlet port to discharge the liquid polymer 11 from the measuring chamber 8 after having carried out the method described above.

In addition, the bottom part 5 comprises a vacuum port 2 connectable to a vacuum generator like a vacuum pump. This port 2 comprises a valve 4, for instance a solenoid valve 4. This vacuum port 2 is used to apply a high vacuum to the measuring chamber 8, for instance in the range of 20 to 100 mbar.

In the embodiment, shown in FIG 2, a further port 3, which is herein after referred to as evacuation port 3, is arranged at an end 7a of the tubular measuring part 7. The evacuation port 3 may be used to apply a low vacuum to the measuring chamber 8, e.g. a vacuum in the range of 800 mbar to 950 mbar, to suck the liquid polymer into the measuring chamber 8. The evacuation port 3 also comprises a valve 4, for instance a solenoid valve.

Alternatively, the liquid polymer could be injected into the measuring chamber 8. By doing so, the evacuation port 3 is opened to allow any air to escape from the measuring chamber.

Moreover, a transparent panel 9 is arranged in the tubular measuring part 7 for observing at least a part of the measuring chamber 8.

Besides, a further transparent panel may be arranged in the cone-shaped chamber part 6 for monitoring the processes going on in the measuring chamber 8. The transparent panel 9 and any the further transparent panel may be configured as a window of any shape and may include any transparent material, for instance glass or acryl glass.

Alternatively, one or more of the tubular measuring part 7, the chamber part 6 and the bottom part 5 may be made of a transparent material for observing the inside of the measuring chamber 8.

A measuring scale 10 may be arranged on the transparent panel 9, for instance printed thereon, for facilitating the measurement of the gas volume, for instance by reading off the height of a free gas column 24 in a tubular measuring part of the measuring chamber or, respectively, by reading off the remaining level of the liquid material.

In the embodiment of the invention, shown in FIG 2, the bottom part 5 comprises a pan-shaped cavity 15 forming a barrier of the measuring chamber 8. The pan-shaped cavity 15 comprises a bottom opening 16 at its lowest point which is connected to the inlet port 1 and to the vacuum port 2 via ducts.

Besides, the bottom part 5 comprises a heating device 14 for heating the liquid polymer 11 in the measuring chamber 8 to reduce the viscosity thereof. Alternatively, the heating device could be arranged in the chamber part 6, for instance in form of a heating coil in the cone-shaped part 6.

The measuring chamber 8 may be made of a metallic material, at least partly, in order to facilitate the heat transfer from the heating device 14 to the liquid polymer 11 in the measuring chamber 8. The metallic material is for instance aluminium, high-grade steel or copper.

Moreover, the apparatus 21 may comprise a sensing device for detecting the filling degree of the measuring chamber 8. Preferably, this sensing device is arranged at the top of the measuring chamber 8, near the evacuation port 3. Furthermore, a pressure sensor may be connected to the measuring chamber 8 to detect the pressure in the measuring chamber 8 for control purposes.

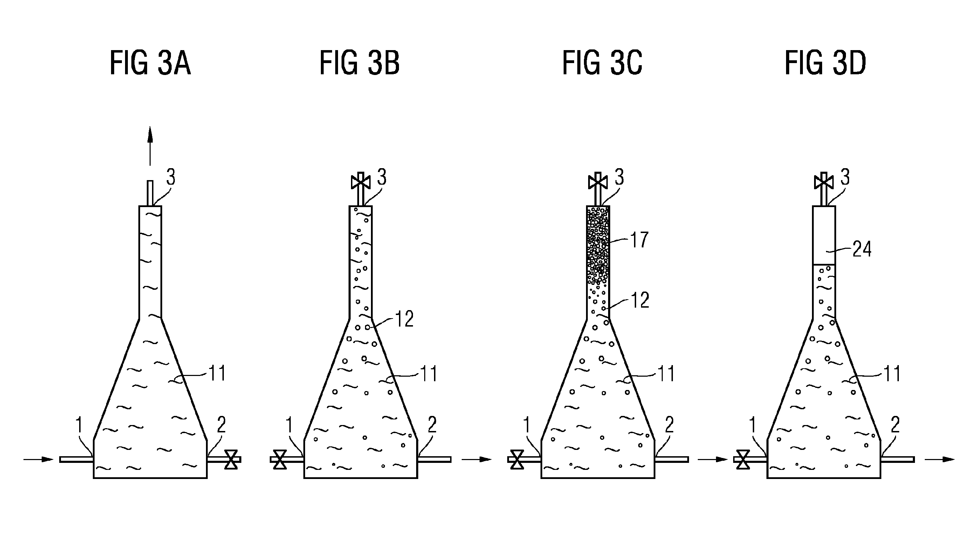

**FIG 3a,** FIG 3b and FIG 3c show schematically how the aforementioned method may be carried out using the apparatus 21, shown in FIG 2.

As indicated by an arrow near the inlet opening 1 in FIG 3a, a liquid polymer material, for instance an epoxide resin, is introduced through the inlet opening 1 into the measuring chamber 8. Preferably, the liquid material 11 is sucked from a container into the measuring chamber 8 by a low vacuum. The low vacuum is applied by a vacuum generator which is connected to the evacuation port 3 at the top of the apparatus 21. This involves filling the measuring chamber 8 completely with the liquid material 11.

Then, as schematically shown in **FIG 3b****,** the inlet opening 1 and the evacuation port 3 are closed. A vacuum generator is connected to the vacuum port 2 and the pressure in the measuring chamber 8 is reduced to a predefined intended pressure. This predefined pressure is below the pressure of the low vacuum applied to the measuring chamber 8 to suck in the liquid material 11. One and the same vacuum generator may be connected sequentially to the evacuation port 3 and to the vacuum port 2.

The volatile impurities are solved out as gas bubbles 12. The gas bubbles 12 coalesce and rise to a free surface of the liquid material 11.

As schematically shown in **FIG 3c****,** a gas bubble column 17 is build-up in the tubular measuring part 7 after a predetermined time.

As further shown in **FIG 3d****,** the gas bubbles collapse after a certain time such that the gas bubble column 17 disappears leaving a certain gas volume in the tubular measuring part 7. Then, the remaining filling level of the liquid material 11 is lower than the filling level at the beginning of the measuring procedure. This remaining filling level or the height of the free gas column 24 in the measuring chamber 8 may be read off by means of a measuring scale on the transparent panel 9.

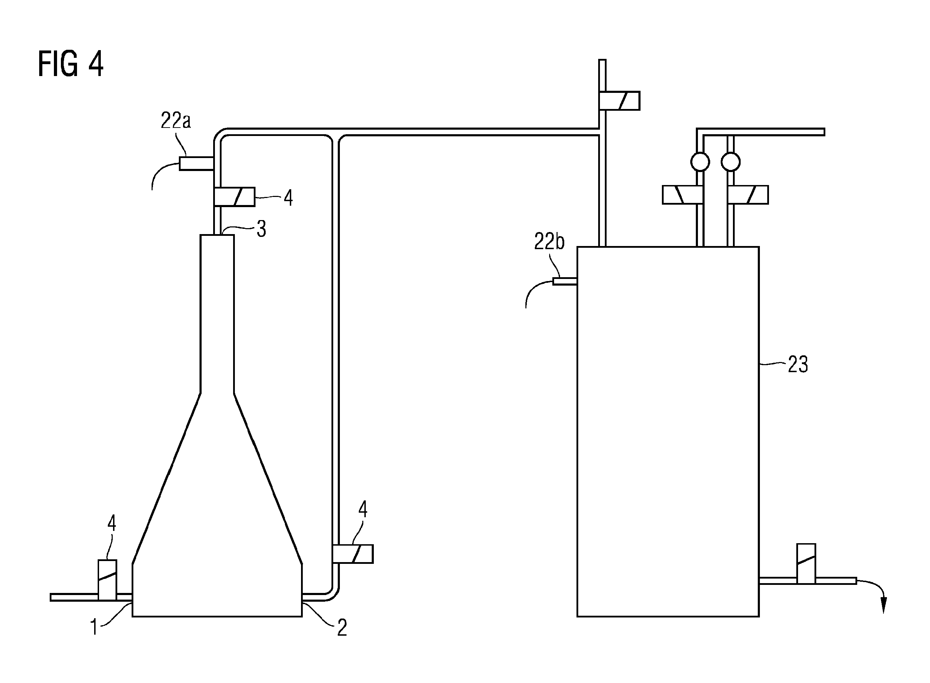

**FIG 4** shows an exemplary measurement setup comprising an apparatus 21 according to an embodiment of the invention, as described above, and a vacuum pump 23. Solenoid valves 4 are connnected to the ports 1, 2 and 3 of the apparatus 21. These are electromechanical valves controlled by an electric current through a solenoid. The solenoid valves offer fast and safe switching, low control power and a compact design. As apparent from FIG 4, one and the same vacuum generator may selectively be connected to the evacuation port 3 and the vacuum port 2. In addition, a sensing device 22a is connected to the evacuation port 3 for sensing the filling level of the measuring chamber 8. A further sensing 22b is connected to the vacuum generator for controlling the vacuum conditions in the measuring chamber 8.

The disclosure is illustrative only and changes may be made in detail, especially in matters of shape, size and arrangement of parts within the principles of the invention in the full extent indicated by the meaning in which the appended claims are expressed.

## Claims

1. Method for determining the amount of volatile impurities in a liquid material comprising the steps of:
- filling (100) a measuring chamber completely with a liquid material comprising volatile impurities,
- closing (110) the measuring chamber hermetically sealed,
- reducing (120) the pressure in the measuring chamber to a predefined pressure such that the volatile impurities are released over a free surface of the liquid material as gas bubbles,
- maintaining the predefined pressure for a predetermined time such that the gas bubbles collapse leaving a gas volume, and
- measuring (130) the gas volume which indicates the amount of volatile impurities in the liquid material.

2. Method according to claim 1, **characterized in that** the step of filling (100) a measuring chamber completely with a liquid material having volatile impurities comprises sucking the liquid material into the measuring chamber.

3. Method according to one of claims 1 or 2, **characterized in that** the liquid material comprises a plastic material.

4. Method according to one of claims 1 to 3, **characterized in that** the liquid material is heated in the measuring chamber to reduce the viscosity of the liquid material.

5. Apparatus (21) for carrying out the method according to one of the claims 1 to 4 with
- a hermetically sealable measuring chamber (8) comprising a bottom part (5), a chamber part (6) and a measuring part (7),
- wherein the bottom part (5) is connected to the chamber part (6) and the chamber part (6) is connected to the measuring part (7) such that the parts (5, 6, 7) enclose the measuring chamber (8) hermetically sealable,
- wherein the bottom part (5) comprises a first closable opening (1) for introducing a liquid material (11) comprising volatile impurities into the measuring chamber (8),
- wherein the bottom part (5) further comprises a second closable opening (2) connectable to a vacuum generator for reducing the pressure in the measuring chamber (8) to a predefined pressure such that the volatile impurities are released over a free surface of the liquid material as gas bubbles (12) which collapse leaving a gas volume, and
- wherein the measuring chamber (8) further comprises a transparent panel (9) for determining the gas volume which indicates the amount of volatile impurities in the liquid material.

6. Apparatus (21) according to claim 5, **characterized in that** the chamber part (6) is at least partly cone-shaped and comprises a tapered end (6a),
wherein the measuring part (7) is a tubular measuring part, and
wherein an end of the tubular measuring part (7a) is connected to the tapered end of the chamber part (6a).

7. Apparatus (21) according to claim 5 or 6, **characterized in that** the measuring part (7) comprises a third closable opening (3) connectable to a vacuum generator for sucking the liquid material (11) through the first closeable opening (1) into the measuring chamber (8).

8. Apparatus (21) according to one of claims 5 to 7, **characterized in that**
the openings (1, 2, 3) are configured as ports, each of which comprises a valve (4), for instance a solenoid valve.

9. Apparatus (21) according to one of claims 5 to 8, **characterized in that**
the bottom part (5) is a cylindrical bottom part (5) which comprises a pan-shaped cavity (15) forming a barrier of the measuring chamber (8), wherein the pan-shaped cavitiy (15) comprises a bottom opening (16) connected to the first closable opening (1) and to the second closable opening (2).

10. Apparatus (21) according to one of claims 5 to 9, **characterized in that**
the bottom part (5) comprises a heating device (14) for heating the measuring chamber (8) to reduce the viscosity of the liquid material.

11. Apparatus (21) according to one of claims 5 to 10, **characterized in that**
the measuring chamber (8) comprises a metallic material.

12. Apparatus (21) according to one of claims 5 to 11, **characterized in that**
the transparent panel (9) comprises a measuring scale (10) for measuring the gas volume.
